# EUROPÄISCHE PATENTSCHRIFT

(11) **EP 3 518 823 B1**
(45) Veröffentlichungstag und Bekanntmachung des Hinweises auf die Patenterteilung: **04.08.2021**
(21) Anmeldenummer: 17784870.2
(22) Anmeldetag: 26.09.2017
(51) Int. Cl.: A61C 17/06, A61B 1/247

(54) **EINWEGABSAUGER**
DISPOSABLE ASPIRATOR
INSTRUMENT D'ASPIRATION À USAGE UNIQUE

(30) Priorität: 28.09.2016 DE 102016011629
(43) Veröffentlichungstag der Anmeldung: 07.08.2019
(73) Patentinhaber: Cleverdent Ltd., 48149 Münster (DE); Clasen, Stephan, 48149 Münster (DE); Kayser, Martin, 50968 Köln (DE)
(72) Erfinder: CLASEN, Stephan, 48149 Münster (DE); KAYSER, Martin, 50968 Köln (DE)
(74) Vertreter: Patentanwälte Bauer Vorberg Kayser
(86) Internationale Anmeldenummer: PCT/EP2017/074357
(87) Internationale Veröffentlichungsnummer: WO 2018/060188

(56) Entgegenhaltungen:
- CH-A- 543 880
- DE-A1-102013 110 302
- DE-B3-102012 100 119
- FR-A1- 2 595 939
- US-A- 4 568 281
- US-A1- 2005 106 527

## Beschreibung

Die vorliegende Erfindung betrifft einen zahnmedizinischen Spiegelsauger zum Absaugen von Flüssigkeiten und Partikeln aus einem Mundraum eines Patienten, mit einem hohlen Grundkörper, der eine Außenfläche, eine Innenfläche, eine Längsachse, eine Anschlussöffnung und eine Ansaugöffnung aufweist.

Bei zahnmedizinischen Behandlungen ist es oftmals notwendig, anfallende Flüssigkeiten oder gelöste Partikel, beispielsweise Speichel, Spraywasser und Blut während der Behandlung abzusaugen. Auch kann Wasser, beispielsweise zum Reinigen oder nach Anwenden einer Multifunktionsspritze anfallen, das abgesaugt werden muss. Hierzu werden üblicherweise Spiegelsauger verwendet, die in der Regel aus einem röhrenförmigen Körper aus Kunststoff gebildet sind, an dessen Ende ein Schlauch befestigt ist, der wiederum mit einer Pumpe verbunden ist. Durch den Schlauch werden die störenden Flüssigkeiten und Festkörper abgeleitet.

Oftmals wird ein Spiegelsauger nicht vom behandelnden Zahnarzt oder Zahnchirurgen selbst, sondern von einer oder einem Assistenten geführt und gehalten, weil der behandelnde Zahnarzt mit der einen Hand ein Bohrwerkzeug und mit der anderen Hand einen Spiegel, über den er den zu behandelnden Bereich einsehen kann, halten muss. Nachteilig bei der beschrieben Vorgehensweise ist, dass die beiden Personen sehr eng beieinander um den zu behandelnden Bereich stehen oder sitzen müssen. Dies kann gerade dann, wenn es sich um verhältnismäßig schwierige oder feinmotorisch anspruchsvolle Eingriffe handelt, für den behandelnden Arzt als störend empfunden werden.

Aus der DE 102006048463 A1 ist ein medizinischer Spiegelsauger bekannt, bei dem die Innenfläche eine durch die Ansaugöffnung sichtbare verspiegelte Oberfläche aufweist. Die erfindungsgemäße spiegelnde Beschichtung ermöglicht es dem Benutzer, den medizinischen Spiegelsauger sowohl als Spiegelsauger zum Abführen von Flüssigkeiten und Partikeln, als auch gleichzeitig als Spiegel zu benutzen. Mit Hilfe eines solchen Spiegelsaugers ist es ihm nun möglich, die Behandlung ohne eine assistierende Person durchzuführen. Der Spiegelsauger wird also gleichzeitig als Spiegelsauger und als Spiegel verwendet. Die Grundidee der Kombination der beiden Geräte ist grundsätzlich sehr gut, allerdings ist die Fertigung aufwändig und mit hohen Kosten verbunden. Hinzu kommt, dass der Aufwand für die Reinigung und Sterilisation der Spiegelsauger ebenfalls hoch ist.

Aus der FR2595939 A1 ist ein Spiegelsauger bekannt, welcher bis auf den eingesetzten Spiegel einteilig ausgestaltet ist.

Die Aufgabe der vorliegenden Erfindung besteht darin, einen gegenüber dem bekannten Spiegelsauger verbesserten Spiegelsauger bereitzustellen. Dieser soll ebenfalls die Möglichkeit bieten den Mundraum während des Absaugens einzusehen. Insbesondere soll der Spiegelsauger kostengünstig herstellbar sein.

Erfindungsgemäß wird die Aufgabe durch einen Spiegelsauger mit den Merkmalen des Patentanspruchs 1 gelöst.

Demnach ist der Spiegelsauger als Einwegspiegelsauger ausgebildet. Somit entfällt die sonst übliche Reinigung und Sterilisation nach der Behandlung, für jeden einzelnen Patienten wird ein neuer Spiegelsauger verwendet.

Um die mit der nur einmaligen Benutzung verbundenen Kosten zu reduzieren, ist der erfindungsgemäße Spiegelsauger aus kostengünstigen Komponenten gefertigt. Insbesondere wird kein sonst üblicher Glasspiegel verwendet. Wesentlich ist, dass der verwendete Spiegel bzw. die spiegelnde Fläche mit geringeren Kosten als ein üblicher Glasspiegel zu fertigen ist.

Gemäß der Erfindung weist ist der Spiegelsauger einen Grundkörper auf, der nur aus einem einzigen Teil besteht. Der Grundkörper ist nur aus einem einzigen Material gefertigt und kann vorzugsweise in nur einem einzigen Fertigungsschritt gefertigt werden. Im Bereich der Ansaugöffnung bildet der Grundkörper eine plane Fläche aus, die mit einem spiegelnden Material versehen ist. Erfindungsgemäß ist die plane Fläche mit Chrom bedampft oder mit spiegelndem Lack lackiert. Somit ist die Herstellung schnell und einfach möglich, die Kosten sind gering.

Gemäß einem Ausführungsbeispiel, welches nicht Teil der beanspruchten Erfindung ist, kann der Spiegelsauger auch aus zwei Grundkörperteilen gebildet sein, die miteinander verbunden, vorzugsweise verschweißt oder verklebt sind und so letzendlich eine quasi-einstückigen Spiegelsauger ausbilden. Quasi-einstückig bedeutet in diesem Zusammenhang, dass die beiden Grundkörperteile nach der Fertigung nur durch Zerstören wieder voneinander getrennt werden können. Besteht der Grundkörper aus zwei Grundkörperteilen, wird zusätzlich ein kostengünstiges Spiegelelement verwendet.

Für die Fertigung eignet sich insbesondere thermoplastischer Kunststoff zum Beispiel Polypropylen oder auch Polyethylen. Durch die Hinzugabe von Zusatzstoffen kann die äußere Erscheinung des Spiegelsaugers beeinflusst werden.

Die Erfindung wird anhand der nachfolgenden Figuren näher erläutert. Diese zeigen lediglich Ausführungsbeispiele, die Erfindung soll nicht auf diese beschränkt sein.

Es zeigen:
- Fig. 1:: einen Spiegelsauger von oben,
- Fig. 2:: den Spiegelsauger aus Fig. 1 von der Seite,
- Fig. 3:: den Spiegelsauger aus Fig. 1 im Längsschnitt,
- Fig. 4:: eine Vergrößerung des Bereichs A aus Fig. 3,
- Fig. 5.:: einen Spiegel in Seitenansicht, wobei ein Spiegelsauger mit separat hergestelltem Spiegel nicht Teil der beanspruchten Erfindung ist,
- Fig. 6:: eine vereinfachte Darstellung des vorderen Bereichs des Spiegelsaugers von unten.

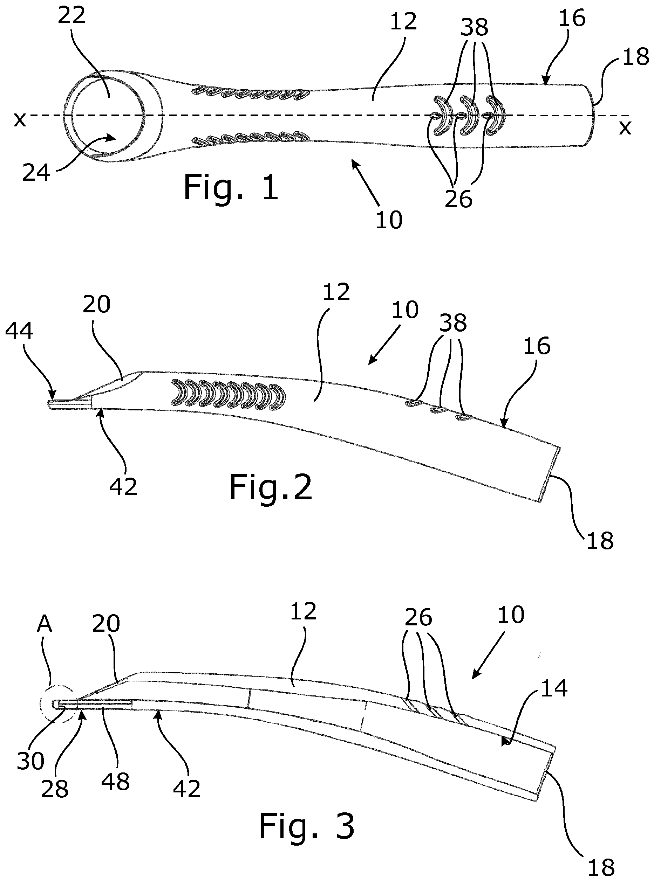

Wie sich insbesondere aus den Figuren 1 bis 3 ergibt, weist der Spiegelsauger 10 einen hohlen rohrförmigen Grundkörper 12 mit einer Innenfläche 14 und einer Außenfläche 16 auf. Weiterhin weist der Grundkörper 12 eine Längsachse X-X auf (vergl. Fig. 1). Die insbesondere in den Figuren 2 und 3 erkennbare Bogenform des Spiegelsaugers 10 hat den Vorteil, dass dieser leichter an die zu behandelnde Stelle herangeführt werden kann.

Der Grundkörper 12 weist eine Anschlussöffnung 18 für einen nicht gezeigten Schlauch und eine Ansaugöffnung 20 zum Ansaugen von Partikeln und Flüssigkeiten auf. Durch die Ansaugöffnung 20 werden die abzusaugenden Flüssigkeiten oder Partikel eingesogen und durch die Anschlussöffnung 18 über den Schlauch abgeleitet.

Innerhalb des Grundkörpers 12 ist ein Spiegelelement 22 im Bereich der Ansaugöffnung 20 angeordnet, das durch die Ansaugöffnung 20 einsehbar ist. Entsprechend ist eine sichtbare Spiegelfläche 24 der Ansaugöffnung 20 zugewandt. Das Spiegelelement 22 ist vollständig innerhalb des Grundkörpers 12, also in Strömungsrichtung der abzusaugende in Luft hinter der Ansaugöffnung 20 angeordnet ist. Die angesaugte Luft wird über die Spiegelfläche 24 geleitet, wodurch ein Beschlagen der Spiegelelementfläche 24 effektiv verhindert wird.

Das Spiegelelement 22 ist vorzugsweise entweder durch eine spiegelnde Metallscheibe oder durch eine Kunststoffscheibe gebildet, die mit einer spiegelnden Folie oder einer anderweitig aufgebrachten Beschichtung versehen ist. Insbesondere kann die Beschichtung durch verdampfen, vorzugsweise mit Chrom, erzeugt werden.

Der Spiegelsauger 10 kann Zusatzöffnungen 26 aufweisen, durch die ebenfalls Luft angesaugte wird. Die Zusatzöffnungen 26 verhindern einen Unterdruck innerhalb des Grundkörpers 12, wenn die Ansaugöffnung 20 beispielsweise durch die Zunge oder Wange des Patienten verschlossen wird. Im gezeigten Ausführungsbeispiel sind drei Zusatzöffnungen 26 vorgesehen, denkbar ist aber auch nur eine einzige Zusatzöffnung 26 oder auch mehr als drei Zusatzöffnungen 26.

Auf der Außenfläche 16 des Grundkörpers 12 sind Profilelemente 38 erkennbar, die einen sicheren Griff des Spiegelsaugers 10 und ein Abrutschen der Finger des behandelnden Zahnarztes verhindern.

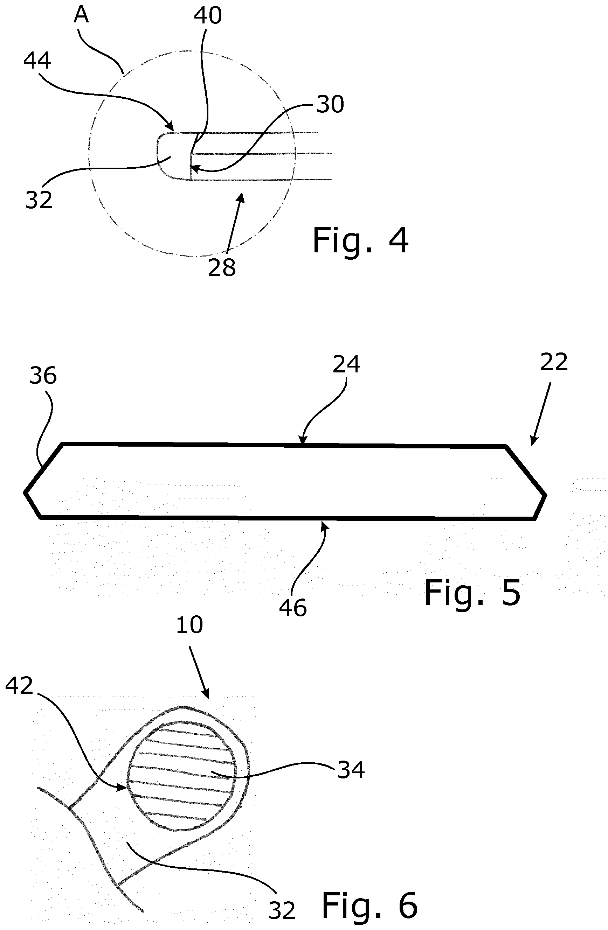

Die Figuren 3 und 6 zeigen dass der Grundkörper 12 aus einem ersten Grundkörperteil 32 und einem zweiten Grundkörperteil 34 gebildet ist. Das erste Grundkörperteil 32 weist eine Spiegelaufnahme 48 mit einer Öffnung 28 auf, in die das Spiegelelement 22 im zusammengesetzten Zustand eingesetzt ist. Eine Innenwandung 30 der Öffnung 28 umgibt das Spiegelelement 22 und liegt an einer Außenwandung 36 des Spiegelelements 22 zumindest bereichsweise an. Die Öffnung 28 verjüngt sich ausgehend von einer Grundkörperunterseite 42 in Richtung der Ansaugöffnung 20. Die Öffnung 28 weist auf ihrer der Grundkörperunterseite 42 zugewandten Seite einen Durchmesser auf der größer als der Durchmesser des Spiegelelements 22 ist. Dies ist Voraussetzung dafür, dass das Spiegelelement 22 in die Spiegelaufnahme 48 einführbar ist.

Das Spiegelelement 22 ist im Querschnitt zumindest abschnittsweise etwa trapezförmig ausgeführt (vgl. Fig. 5), so dass sein Durchmesser zumindest abschnittsweise ausgehend von das Spiegelelementfläche 24 in Richtung einer Grundkörperunterseite 42 zunimmt. Das Spiegelelement 22 weist eine der sichtbaren Spiegelfläche 24 abgewandte Spiegelrückseite 46 auf. In Fig. 5 ist ein Ausführungsbeispiel gezeigt, bei der das Spiegelelement 22 in vertikaler Richtung etwa im unteren Drittel einen maschinellen Durchmesser aufweist. Diese Form vereinfacht das Einsetzen oder Einklipsen in die Spiegelaufnahme 48. An den unteren Bereich der Außenwandung 36 liegt eine untere Halteschulter an.

Fig. 4 verdeutlicht in einer vergrößerten Darstellung des Bereichs A aus Fig. 4, dass eine obere Halteschulter 40 die gesamte Außenwandung 36 des Spiegelelements 22 umgibt, und einen Raum neben und unterhalb des Spiegelelements 22 abdichtet. Die Abdichtung wird über eine Vorspannung der oberen Halteschulter 40 verbessert. Dies bedeutet, dass das Spiegelelement 22 beim Einsetzen in das erste Grundkörperteil 32 gegen die obere Halteschulter 40 gedrückt wird und diese minimal komprimiert oder elastisch verformt wird.

Weiterhin hat diese Ausführungsvariante den Vorteil, dass das Spiegelelement 22 nahezu bündig in eine Bodenfläche 44 eingelassen ist.

Fig. 6 zeigt den vorderen Bereich des Spiegelsaugers 10 mit Sicht auf die Grundkörperunterseite 42. Erkennbar ist das zweite Grundkörperteil 34 (schraffiert), welcher die Öffnung 28 verschließt.

## Patentansprüche

1. Zahnmedizinischer Spiegelsauger (10) zum Absaugen von Flüssigkeiten und Partikeln aus einem Mundraum eines Patienten, mit einem rohrförmigen hohlen Grundkörper (12), der eine Innenfläche (14), eine Außenfläche (16), eine Längsachse (X-X), eine Anschlussöffnung (18) für einen Schlauch und eine Ansaugöffnung (20) aufweist, wobei die Innenfläche (14) eine durch die Ansaugöffnung (20) einsehbare verspiegelte Oberfläche (24), aufweist, die derart angeordnet ist, dass der Mundraum über diese zumindest bereichsweise einsehbar ist, wobei
- der Spiegelsauger (10) als Einwegspiegelsauger ausgebildet ist,
- der Grundkörper (12) nur aus einem einzigen Teil besteht,
und
- der Grundkörper nur aus einem einzigen Material gefertigt ist, **dadurch gekennzeichnet, dass**
- der Grundkörper (12) im Bereich der Ansaugöffnung (20) eine plane Fläche ausbildet, die mit einem spiegelnden Material versehen ist, wobei die plane Fläche mit Chrom bedampft oder mit spiegelndem Lack lackiert ist.

## Claims

1. A dental mirror suction device (10) for suctioning liquids and particles from an oral cavity of a patient, with a tubular hollow main body (12) having an inner surface (14), an outer surface (16), a longitudinal axis (X-X), a connection opening (18) for a hose, and a suction opening (20), wherein the inner surface (14) has a mirror-coated surface (24) which is viewable through the suction opening (20) and which is arranged such that the oral cavity is viewable at least in some portions via said mirror-coated surface, wherein
- the mirror suction device (10) is configured as a disposable mirror suction device,
- the main body (12) consists only of a single part, and
- the main body is produced only from a single material, **characterised in that**
- the main body (12), in the region of the suction opening (20), forms a planar surface which is provided with a reflective material, wherein the planar surface is vapour-coated with chromium or is painted with a reflective paint.

## Revendications

1. Aspirateur dentaire à miroir (10) pour aspirer des liquides et des particules de la cavité buccale d'un patient, comprenant un corps de base (12) creux tubulaire qui présente une face intérieure (14), une face extérieure (16), un axe longitudinal (X-X), une ouverture de raccordement (18) pour un flexible et une ouverture d'aspiration (20), dans lequel la face intérieure (14) présente une surface (24) réfléchie pouvant être vue à travers l'ouverture d'aspiration (20), qui est disposée de façon à ce que la cavité buccale puisse être vue au moins par tronçons par le biais de celle-ci, dans lequel
- l'aspirateur à miroir (10) est conçu en tant qu'aspirateur à miroir à usage unique,
- le corps de base (12) est composé d'une seule partie,
et
- le corps de base est fabriqué dans un seul matériau, **caractérisé en ce que**
- le corps de base (12) forme au niveau de l'ouverture d'aspiration (20) une surface plane qui est dotée d'un matériau réfléchissant, dans lequel la surface plane est métallisée au chrome ou vernie au vernis réfléchissant.
